# EUROPEAN PATENT SPECIFICATION

(11) **EP 1 575 665 B1**
(45) Date of publication and mention of the grant of the patent: **29.11.2006**
(21) Application number: 03812750.2
(22) Date of filing: 02.12.2003
(51) Int. Cl.: A61N 1/36, A61N 1/37, A61N 1/368, A61N 1/08

(54) **EXTERNALLY ACTIVATED NEURO-IMPLANT WHICH DIRECTLY TRANSMITS THERAPEUTIC SIGNALS**
THERAPEUTISCHE SIGNALE DIREKT ÜBERTRAGENDES EXTERN AKTIVIERTES NEUROIMPLANTAT
NEURO-IMPLANT ACTIVE DE L'EXTERIEUR, QUI TRANSMET DIRECTEMENT DES SIGNAUX THERAPEUTIQUES

(30) Priority: 12.12.2002 TR 200202651
(43) Date of publication of application: 21.09.2005
(73) Proprietor: Tulgar, Metin, 34860 Kartal- Istanbul (TR)
(72) Inventor: Tulgar, Metin, 34860 Kartal- Istanbul (TR)
(74) Representative: Cayli, Hülya
(86) International application number: PCT/TR2003/000092
(87) International publication number: WO 2004/052450

(56) References cited:
- EP-A- 1 166 820
- US-A- 4 361 153
- US-A- 4 741 339
- US-A- 5 070 535
- US-A- 6 088 619
- US-B1- 6 493 587

## Description

An externally powered and controlled neuro-implant system for transmission of stimulating therapeutic signals to an implantable electrode. The system basically consists of two coils, one external active coil and one internal passive coil each housed in a ferrite pot core which enhances inductive coupling and minimizes the coils in size, thus facilitating the construction of a passive coils array to enable the usage of multi-contact electrodes for swithching of the electrical stimulation between a number of sites along the target neurons. The implanted part of the system, comprising only a coil housed in a ferrite pot core, is fully passive. The passive coil, that is implanted under the skin, is connected with the electrode placed in neighbouring of the target neural tissue via implanted thin medical grade wires. The active coil is placed on the skin overlying the passive coil. Therapeutic signals produced by the transmitter outside the body are transmitted through the coils by inductive coupling across the skin of the patient.

### BACKGROUND OF THE INVENTION

The present invention relates to an externally activated signal transmission system for especially implantable neurostimulators. In the relevant medical literature, such devices are called neuro-implant. Neuro-implant is a device that electronically stimulates the nerves system. Neurostimulation is a process, by which nerves partially loosing their function as a result of disease or travma, are stimulated using artificial electrical pulses for regeneration. Electrical signals used for this purpose must be consistent with the natural activity of human neurophysiology.

Implanted electrical stimulators were first used in 1967. They were primarily developed as a spinal cord stimulator for the management of chronic pain. In the case of persistent and extensive pain, transcutaneous stimulation is not adequate due to need for multiple electrode placement and increased skin impedance. In order more effectively to cover the painful area, direct stimulation of the spinal cord is necessary via an implantable electrode system. Further clinical studies showed that, in addition to control of pain, this method could also be effective on other conditions, e.g. epidural spinal cord stimulation for the treatment of peripheral vascular disease in the lower extremities and angina pectoris, movement disorders with partial motor problems, vagus nerve stimulation for the management of epilepsy, phrenic nerve stimulation for diaphramme pacing in respiratory disorders, deep brain stimulation for the management of parkinson's disease, peroneal nerve stimulation for gait correction in hemiplegic patients with dropped foot, cohlear implant for improving hearing in patients with heavy hearing losses.

The existing implantable neurostimulators operate using either radio-frequency (RF) transmission and fully implantation technics. RF based implants have four components: transmitter, antenna, receiver, and electrode. The transmitter and antenna are external components; the receiver and the electrode are internal components that are implanted in the body by the surgeon. The transmitter, powered by a 9 volt battery, generates RF signals by which electrical impulses are carried. The frequency of carrier waves is about 2 MHz, chosen to minimize the possibility of interference from outside sources, including microwave ovens and amplitude modulated (AM) and frequency modulated (FM) radios. These radio-waves are relayed, via the external antenna, through the skin to the receiver. The passive receiver then translates these signals into electrical impulses and deliveres them to the electrode implanted on the target nerve tissue, insulated stainless-steel wires. Totally implantable systems with long-life lithium battery evantually needs to be replaced by another surgical procedure at approximately 5 years intervals.

A fundamental requirement for successful neuroimplantation is to deliver and maintain effective stimulation to the appropriate nerves, stimulation paraesthesia must cover completely the area of target neurons, and it must not trigger unwanted segmental sensations. There are many reports about the successful use of neuroimplants which have been around for 38 years, but some complaints about their performance were also voiced. The problems encountered with the present implantable stimulator systems can be classified as follows: 1) Breakdown in the electronic components: The existing RF implants relay on the implantation of a receiver circuit which include miniature electronic components and, as component failure is not unknown, patients can be subjected to further surgery to replace a defective receiver. 2) Expiration of battery: totally implantable devices powered by a long-life battery that eventually needs replacement at approximately 5 year intervals, apart from component failure, also require extra surgery to replace the used battery. 3) Programming difficulties: patients wearing a fully implantable system have to go to hospital at certain times for the arrangement of stimulation parameters, and sometimes there are difficulties in externally programming the implanted circuit. 4) Fixed electrical parameters: majority of the existing systems, once implanted, generate a fixed electrical output preset by the manufacturers. The stimulation mode is of a conventional type that composes of pulses with constant frequency preset by the manufacturer. Some of the most sophisticated systems do allow variations of some parameters, but this facility is both limited and expensive. 5) Electrode position: during the operation the electrode may be misplaced or, following the operation, electrodes may migrate, thus reducing the efficacy of stimulation. Multi-contact electrodes have been produced to solve this problem. 6) The expense of the equipment: the high cost of the present implants severely limits widespread use of this clinically approved method.

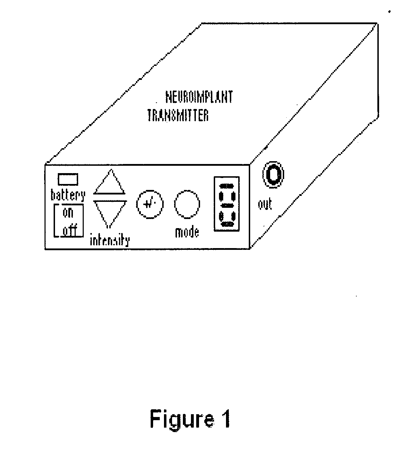

To overcome the problems mentioned above, the present neuro-implant system, based on principle of trans-dermal inductive coupling through one external and one internal coil each housed in a ferrite pot core, has been developed (FIGS. 1, 2, 3 and 4).

While the use of magnetic coupling principles in numerous electromagnetical devices (e.g. transformers), electromagnetic coils housed in a ferrite pot core have not previously been used in neuroimplantation and other implantable medical devices. There are implantable bone healing stimulators making use of rod shaped ferrite cores, but these systems are intended for the transmission of radio-frequency signals which is also common in transistor radio-circuits.

Document WO.98/09588 discloses a system for providing transdermal communication with, and power to, a device implantable into the body of the patient.

Cardiac pace-maker implants operating with inductive coupling principles, which were developed by Abrams and his colleagues in 1960, and applied by Irish cardiologists, Neligan and Malley in 1971, involve in air cored coils which are bigger in size (55 mm in diameter). A big implant is not surgically preferrable. In the present system, the coils are housed in a ferrite pot core; this does not only enhance the inductive coupling but, more importantly, allows a 79 % reduction in size compared with the original cardiac pacemaker coils (FIGS. 21 and 24). The ferrite pot cores used for housing the coils of the present system also facilitate fabrication of passive coils array to compact use of the system with multi-contact electrodes, thus combat some of the difficulties of placement and targeting neurons for long term effective electrical stimulation therapy (FIG. 27).

### SUMMARY OF THE INVENTION

A general object of the present invention is to provide an improved system and method for the transmission of therapeutic stimulating signals to an electrode implanted in the body. Implantable neurostimulators including spinal cord stimulator implant, vagal stimulator implant, diaphramme pacing implant, deep brain stimulator implant, gait corrector implant and cochlear implant are especially suitable to employ this system. Other implantable medical devices can also utilize such a system for therapy or recording with respect to the brain, spinal cord, nerves, muscles, bones, or other tissue or body organs.

The system mainly consists of four elements: two coils, one passive coil and one active coil, an electrode and a transmitter. The passive coil and electrode are internal components implanted in the body; the active coil and transmitter are external. The passive coil is connected to the electrode via insulated thin wires. The active coil is then placed on the skin overlying the implanted passive coil. Therapeutic signals produced by the transmitter are linked to the active coil by means of a flexible cable, and are transmitted through the coils by inductive coupling across the skin of the patient. Each coil is housed in a ferrite pot core that enhances inductive coupling, and minimizes the size of coils thus facilitating the construction of a passive coils array to use multi-contact electrodes for effectively selecting the target neurons.

The main goal of the present invention is that the implanted part of the system is completely passive, having only a coil housed in a ferrite pot core, thus eliminating the risk of additional surgery due to electronic breakdown or battery replacement.

Other objects and advantages of the present system are given in the following detailed description of the invention.

### DESCRIPTION OF THE INVENTION

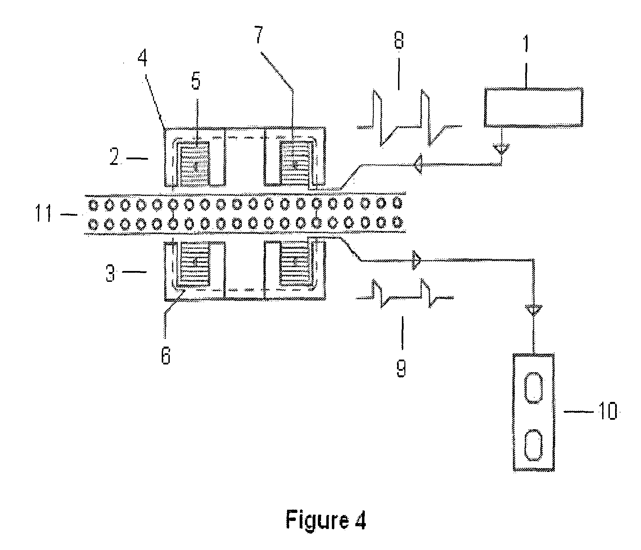

This invention relates to an externally powered and controlled signal transmission system [(1),(2),(3),(4),(5),(6),(7),(8),(9),(10),(11)] for implantable medical devices, particularly neuro-implants, e.g. spinal cord stimulator implant to control pain and to treat vascular diseases such as peripheral vascular disease in lower extremities and coronary arterial disease, angina pectoris and motor disorders, vagus nerve stimulator implant for the management of epilepsy, phrenic nerve stimulator implant for diaphramme pacing in respiratory disorders, deep brain stimulator implant for the management of parkinson's disease, peroneal nerve stimulator implant for gait correction of dropped foot in hemiplegy, cohlear implant for improving hearing (FIGS. 1 and 4).

The device is essentially two electromagnetic coils [(2),(3)] - a passive coil (3) and an active coil (2)- through which electrical signals for neurostimulation are transmitted by trans-dermal inductive coupling (FIGS. 1 and 4). The passive coil (3), that is implanted under the skin, is connected with the electrode (10) located in neighbouring of the target nerve tissue (FIGS. 1, 2 and 4). The active coil (2) is then placed on the skin overlying the implanted passive coil (FIGS. 1, 3 and 4). Therapeutic signals (8) produced by a transmitter device (1) outside the body are linked to the active coil (2) via a flexible insulated cable, and are transmitted through the coils [(2),(3)] across the skin (11).

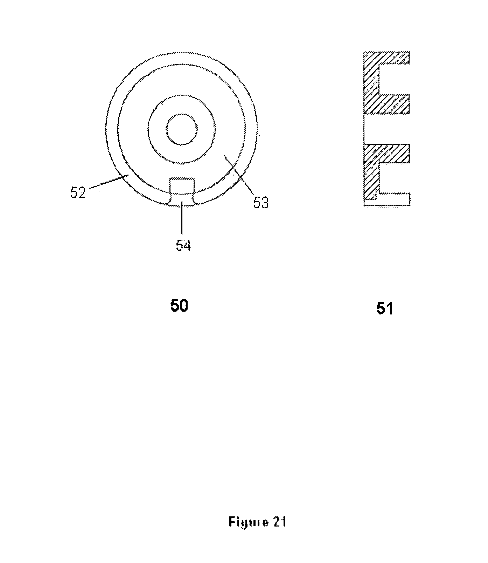

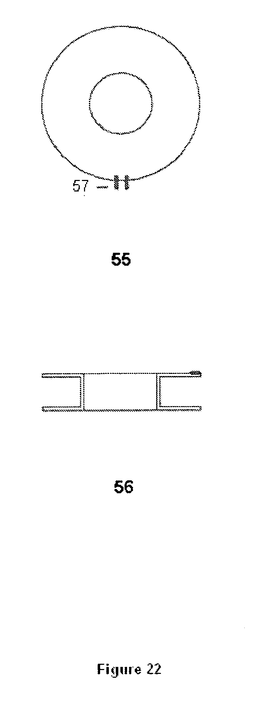

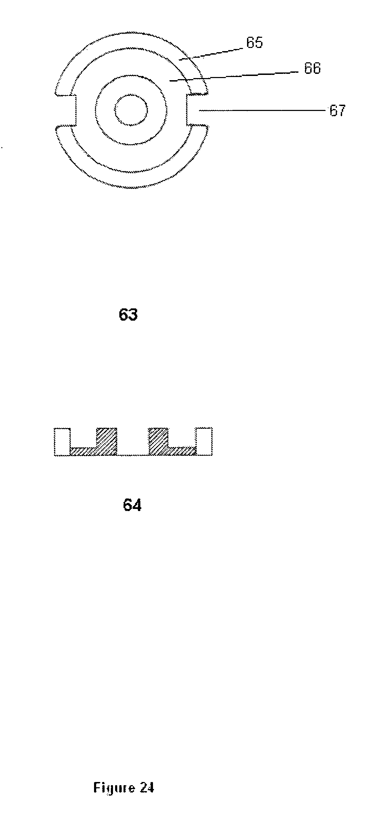

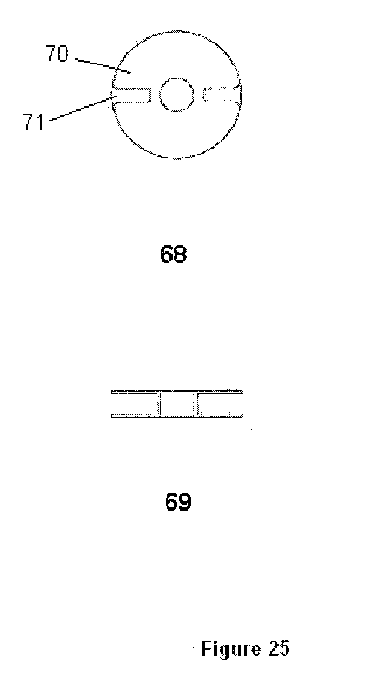

Both coils are formed by wrapping 42 S.W.G. (standart wire gauge) enammelled copper wire [(5),(85)] on bobbins (coil formers) [(55),(56),(57),(66),(67),(69),(70),(71),(72),(73),(74)] made from food grade acetal, delrin (FIGS. 22 and 25). The number of turns of active coil (3) is 1100, and that of passive coil (3) 1000, or the number of turns of both coils can be arranged as any suitable numbers in accordance with application field of therapy. Then each bobbin is placed in a circular ferrite pot core [(4),(52),(65)] (FIGS. 21 and 24).

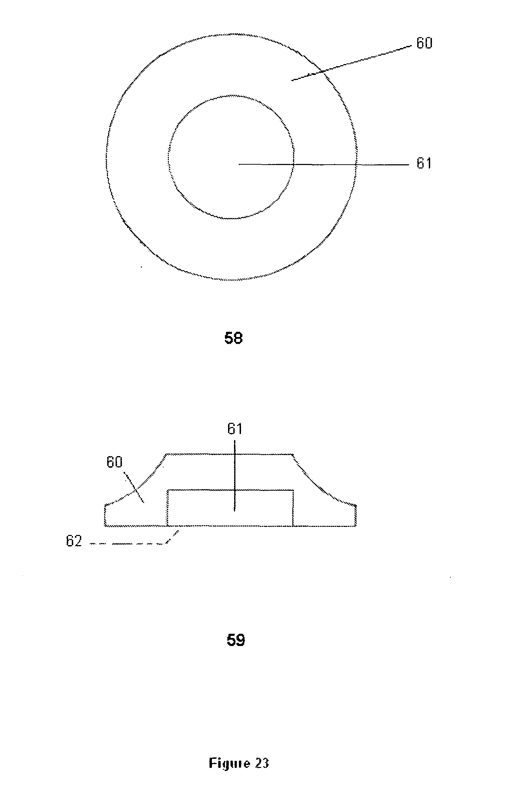

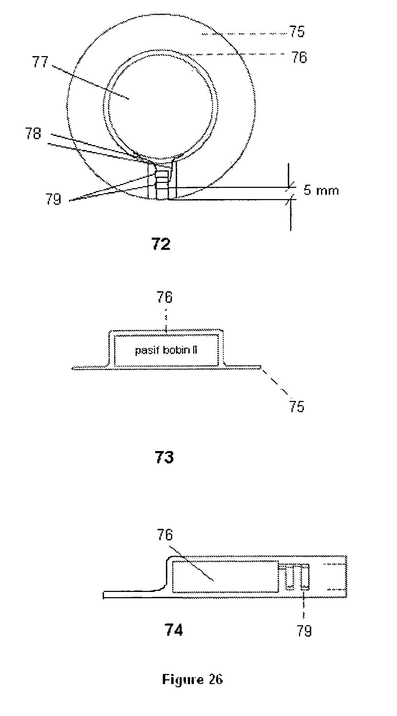

The internal passive coil (3) and its connector are hermetically encapsulated by medical grade materials such as silicone, elastomer, adhesive, polyurethane or titanium [(78),(87),(60)] (FIGS. 23 and 26).

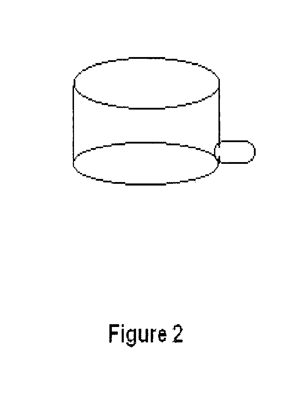

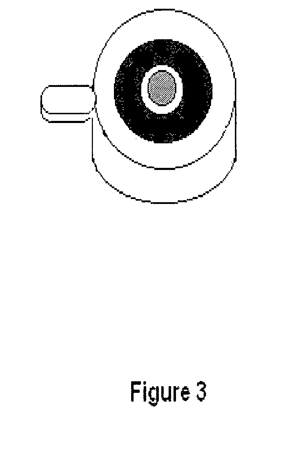

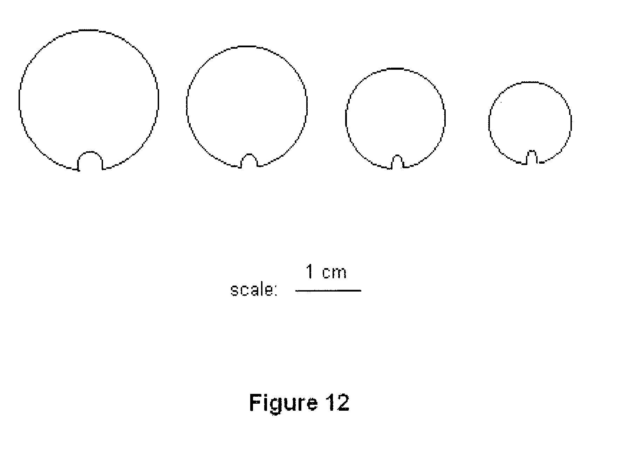

Housing each of the external (2) and internal coils (3) in a ferrite pot core [(4),(52),(65)] enhances inductive coupling and miniaturisation of the system. The external active coil [(2),(4),(52),(65)] is bigger in size (29 mm in diameter, 9 mm in height) to keep the coupling efficiency against lateral movements [(48),(49)] over the implanted passive coil (3) (FIGS. 3, 13 and 14). The internal coil [(3),(80),(88)] is small enough in size (14.4 mm in diameter, 7.5 mm in height) (FIGS. 2 and 12); two or three of them can be used together to form a passive coils array to enable the use of multi-contact electrodes (FIGS. 21, 24 and 27). The only thing the patient need to do is to move the single active coil (2) over the passive coils array [(83),(84)] to select the most effective channel of the multi-contact electrode for switching of electrical stimulation between a number of sites, and thereby combat some of the difficulties of placement, targeting and accommodation (FIG. 1).

In the present system, the implanted part comprising only a coil (3) housed in a ferrite core [(4),(52),(65)] is fully passive (FIGS. 2 and 4); therefore, extra surgery due to component failure or to replace the battery is unlikely, and patients can use such a system as long as they need.

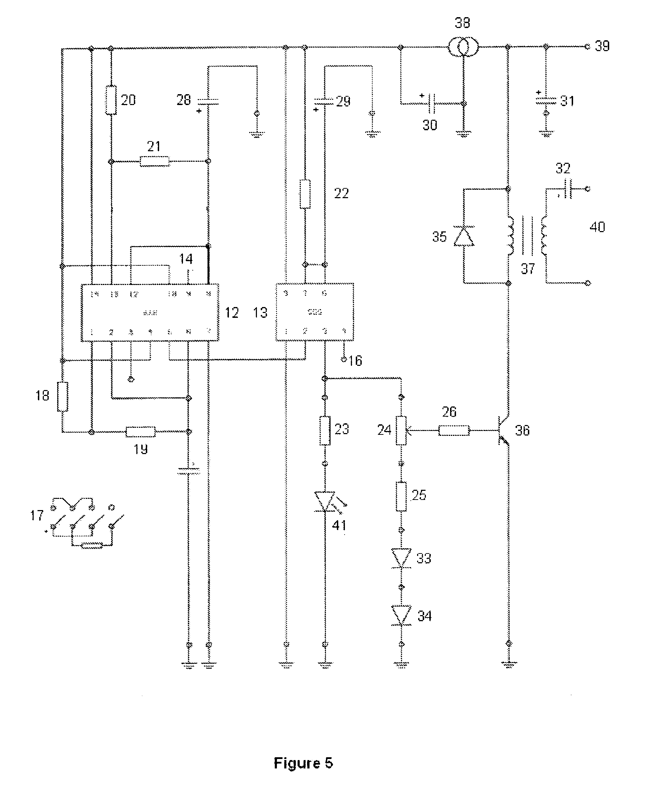

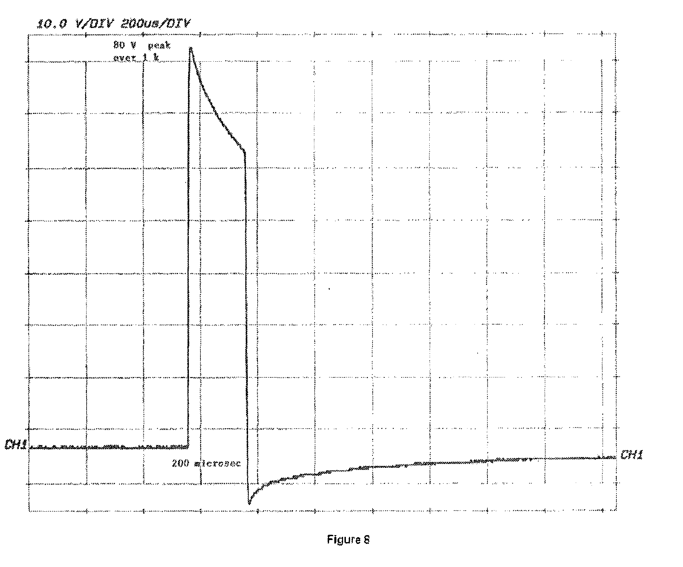

The transmitter circuit of the present system (FIG. 5) has less number of electronic components
(12),(13),(14),(15),(16),(17),(18),(19),(20),(21),(22),(23),(24),(25),(26),(27),(28),(29), (30),(31),(32), (33),(34),(35),(36),(37),(38),(39),(40),(41),(42)] than those of even common portable transcutaneous electrical nerve stimulator (TENS) devices. It is, therefore, a cheaper and more reliable device. On the other hand, it is a versatile system providing all form of electro-therapeutic signals including conventional stimulation (in this mode; continuous pulses are repeated at a constant frequency between 30 Hz and 100 Hz), the burst (in this mode; 80 ms long trains of pulses with an internal frequency of 80 Hz are repeated 1.3 times a second, each train consisting of 7 pulses) and frequency modulated stimulation patterns (in this mode; fast pulses (110 Hz) are slowed down (55 Hz) for a short period (90 ms) 1.3 times a second, and then they get faster again), that are known to be more effective in some clinical conditions, with externally easy programming (FIGS. 8, 9, 10 and 11).

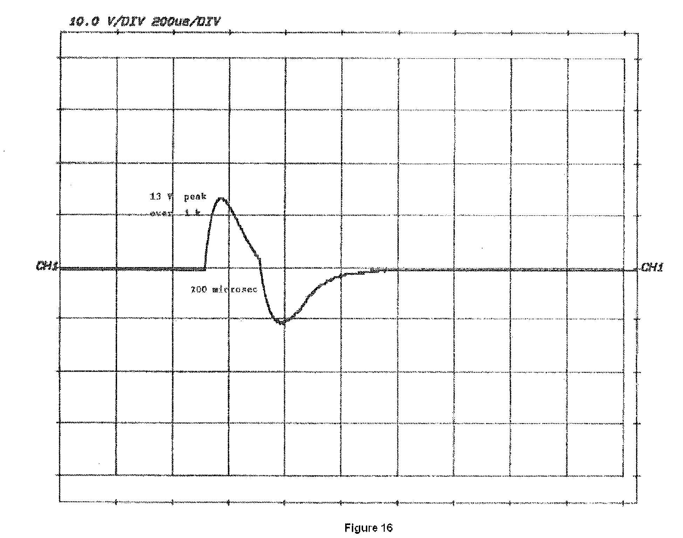

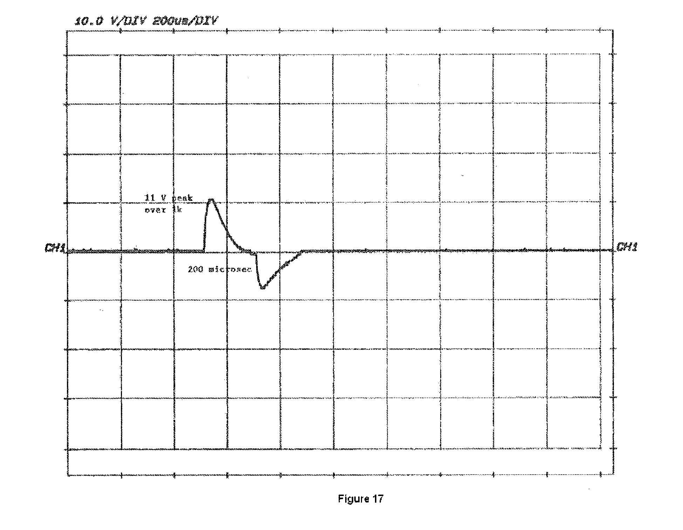

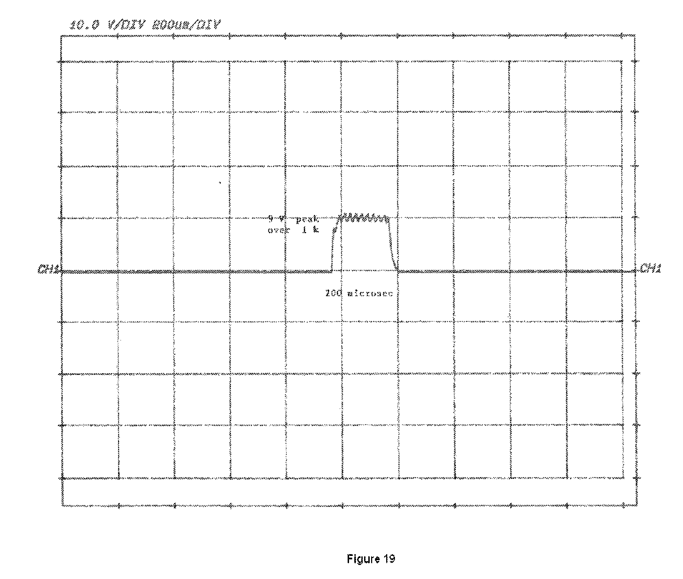

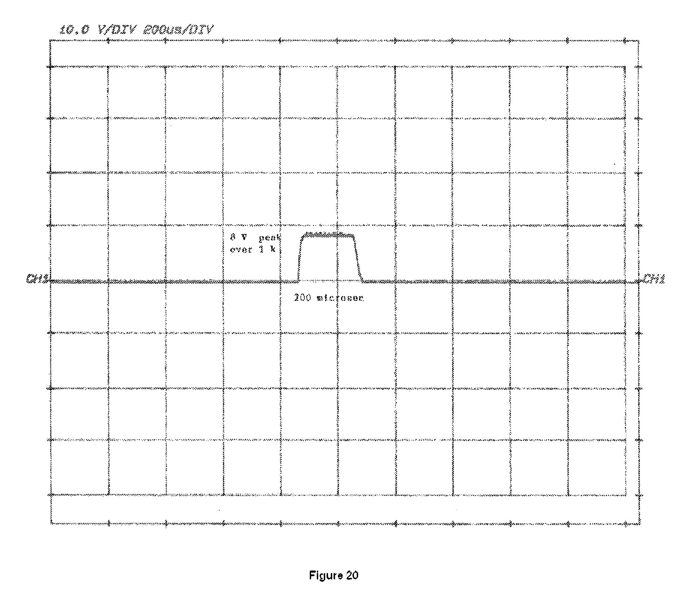

The signal transmitted by the existing RF and totally implantable devices is monophasic (FIGS. 19 and 20) which means involment of direct current (DC). Electrolysis resulting from the polarity is a known factor to be considered. The pulse induced by the present system is biphasic DC free signal (FIGS. 16 and 17) which is useful to minimize any undesirable electrolysis phenomena that may result in breakage in the lead of electrode and tissue necrosis.

All these factors convey the additional advantages of safety and reliability while reducing the cost.

For patients' safety, as demonstrated by a series of environmental tests close to an electricity mains, a microwave oven, a television, a high voltage transformer station, and under a high voltage energy transmission line, accidental induction or interference in the induced pulse patterns is unlikely.

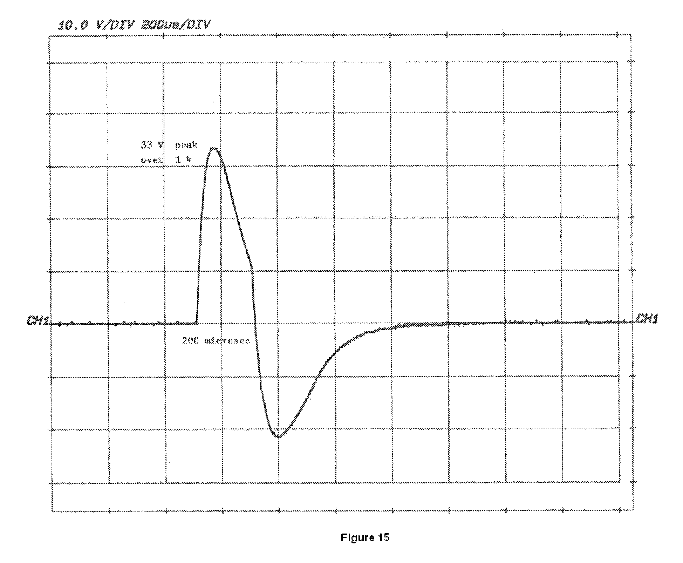

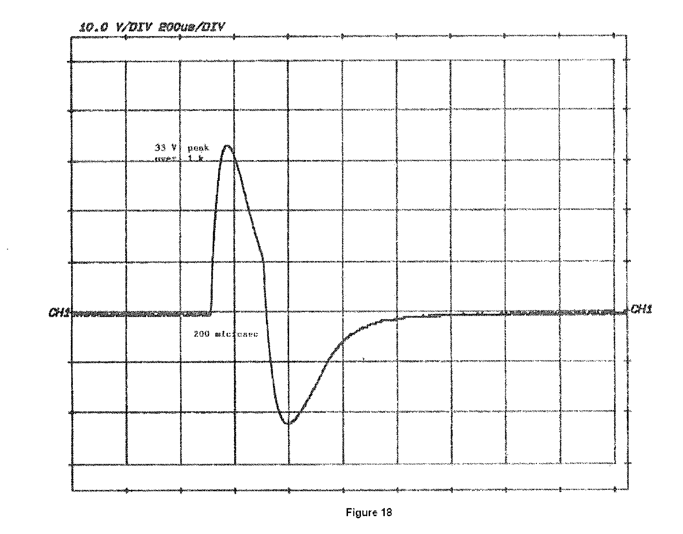

A slight increase in the resistance of passive coil (3) at body temperature (37 °C) in accordance with the known principles of electrotechnics, makes no change in shape and amplitude of the induced pulse (40) (FIGS. 15 and 18).

### BRIEF DESCRIPTION OF THE DRAWINGS

**FIG. 1.** is a general view of the present neuro-implant system.
**FIG. 2.** is a general view of the internal passive coil.
**FIG. 3.** is a general view of the external active coil.
**FIG. 4.** is a schematic illustration of the transmission of therapeutic pulses by inductive coupling through the skin.
**FIG. 5.** shows circuit diagram of the transmitter which drives the external active coil. "Circuit components has been described in section called.

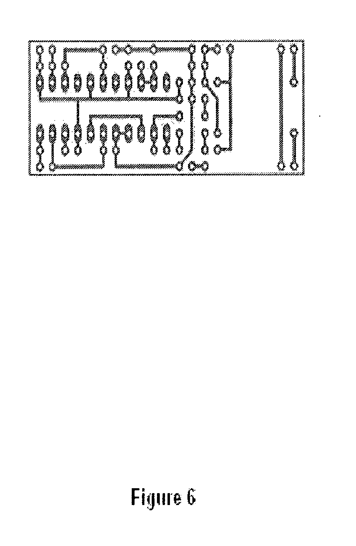

**FIG. 6.** shows printed circuit board (pcb) for the placement of the electronic components of the transmitter (scale: 1/1).

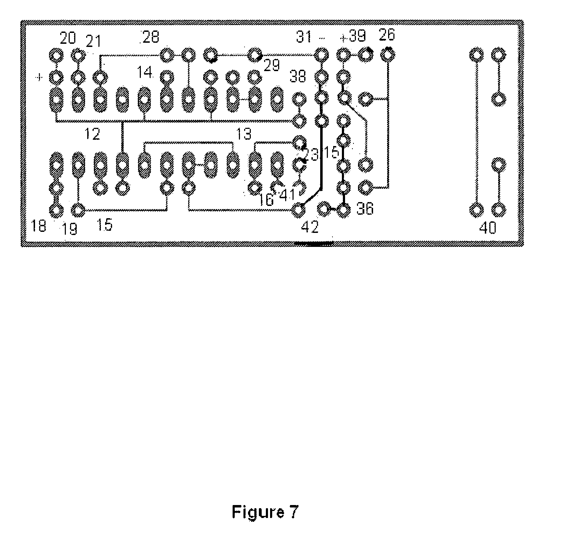

**FIG. 7.** shows enlarged pcb of the transmitter for the location of components (scale: 2x1).
**FIG. 8**. is a single pulse produced by the transmitter in all stimulation modes. Pulse shape: asymetric biphasic rectangular, pulse width: 200 µs (can be selected between 50 µs and 400 µs by changing value of resistor R5 in the transmitter circuit), amplitude: 80 V over 1 kΩ (80 mA).

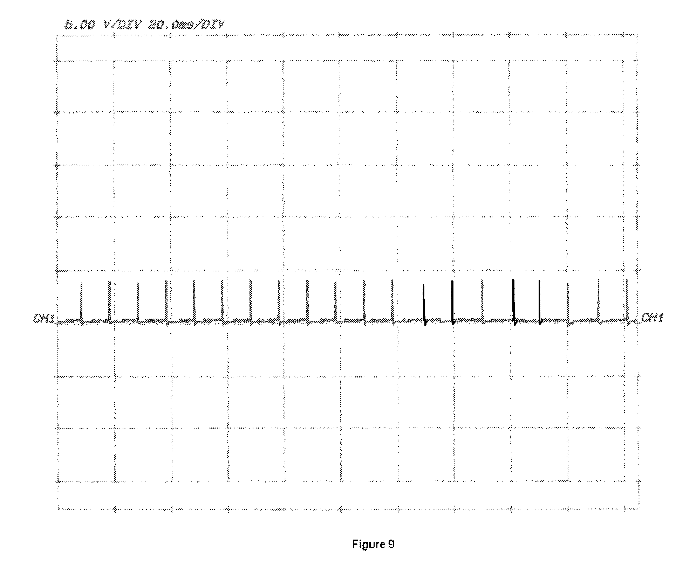

**FIG. 9.** shows pulse patterns produced by the transmitter when set for the conventional mode of stimulation. In this mode; continuous pulses are repeated at a constant frequency between 30 Hz and 100 Hz.

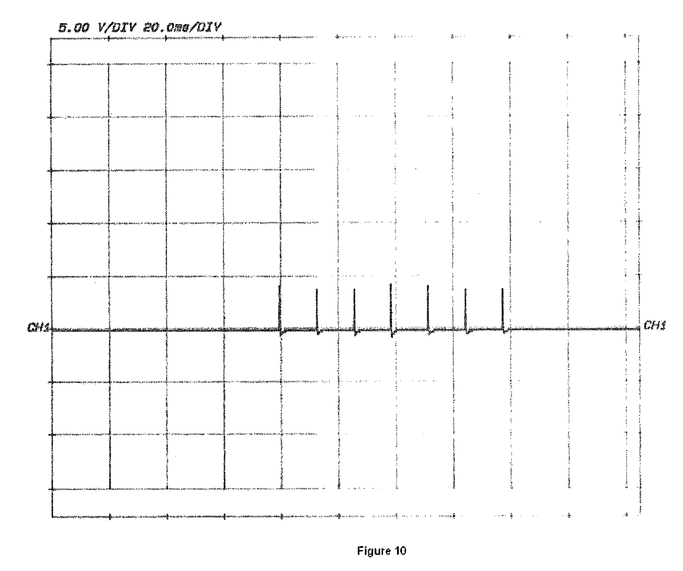

**FIG. 10.** shows pulse patterns produced by the transmitter when set for the burst mode of stimulation. In this mode; 80 ms long trains of pulses with an internal frequency of 80 Hz are repeated 1.3 times a second, each train consisting of 7 pulses. The number of pulses in each train, internal frequency and repeatation rate of the trains can be selected as wanted by changing the values of the relevant components in the transmitter circuit.

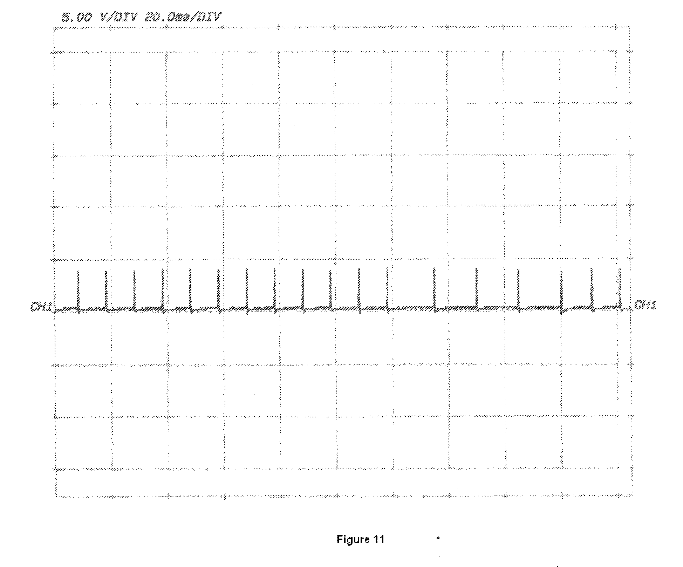

**FIG. 11.** shows pulse patterns produced by the transmitter when set for the frequency modulated stimulation. In this mode; continuous pulses fluctate between 110 Hz and 55 Hz over 60 ms, 1.3 times a second. Fast pulses (110 Hz) are slowed down (55 Hz), for a short period (90 ms) 1.3 times a second, and then they get faster again. The frequency of fast and slow pulses can be selected as wanted by changing the values of the relevant components in the transmitter circuit.
**FIG. 12.** shows the coils tested to select optimal size for the active and passive coils (from left to right, the first one is the active coil, and the others passive).

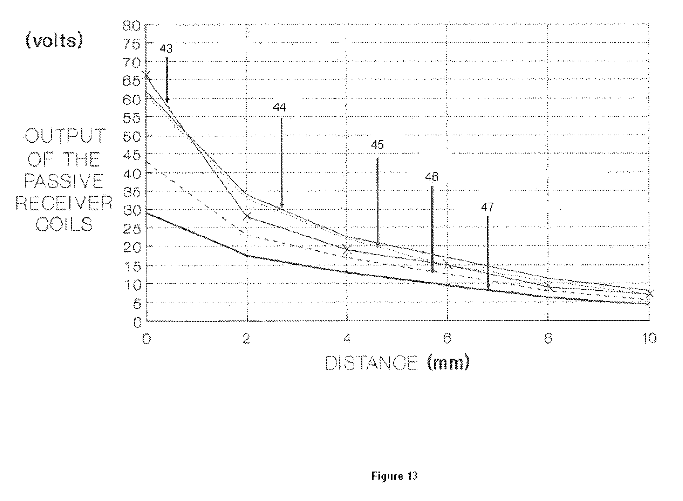

**FIG. 13.** is graphical representation of the vertical distance tests using active and passive coils.

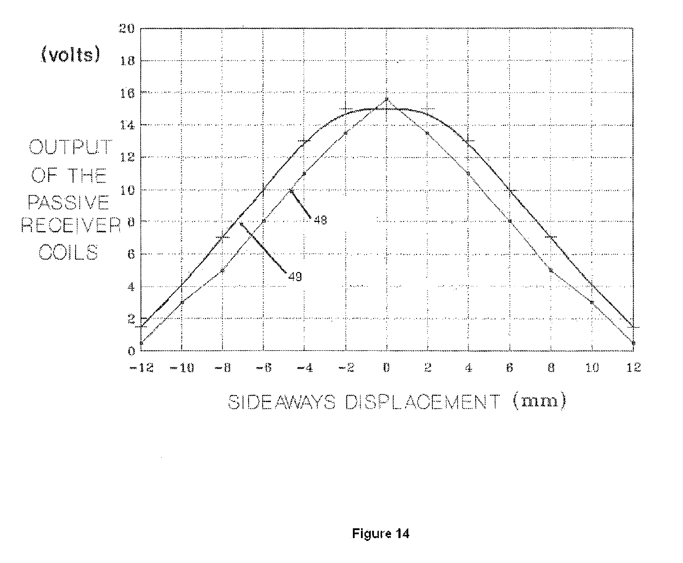

**FIG. 14.** is graphical representation of the lateral distance tests using active and passive coils.
**FIG. 15.** shows output signal of the passive coil (I) when the active coil is placed right on it.
**FIG. 16.** shows output signal of the passive coil (I) when separated from the active coil by 5 mm thick pig skin.
**FIG. 17.** shows output signal of the passive coil (II) when separated from the active coil by 5 mm thick pig skin.
**FIG. 18.** shows output signal of the passive coil (I) operating at 37°C when the active coil is placed right on it.
**FIG. 19.** shows output signal of a commercially available spinal cord stimulator implant (Medtronic, model: 3521) when separated from the transmitter aerial by a vertical distance of 5 mm). "Amplitude: 9 mA, pulse width: 200 µs, pulse shape: monophasic rectangular.
**FIG. 20.** shows output signal of a commercially available spinal cord stimulator implant (Avery, model: S-218) when separated from the transmitter aerial by a vertical distance of 5 mm). "Amplitude: 8 mA, pulse width: 200 µs, pulse shape: monophasic rectangular. This result shows that it includes direct current (DC) component which is not wanted during physical therapy".
**FIG. 21.** is technical drawing of the ferrite pot core used for housing active coil (top view and cross section).
**FIG. 22.** is technical drawing of the coil former used for active coil (top view and cross section).
**FIG. 23.** is technical drawing showing encapsulation of the active coil (top view and cross section).
**FIG. 24.** is technical drawing of the ferrite pot core used for housing passive coil (top view and cross section).
**FIG. 25.** is technical drawing of the coil former used for passive coil (top view and cross section).
**FIG. 26.** is technical drawing showing encapsulation of the passive coil (top view and cross section).

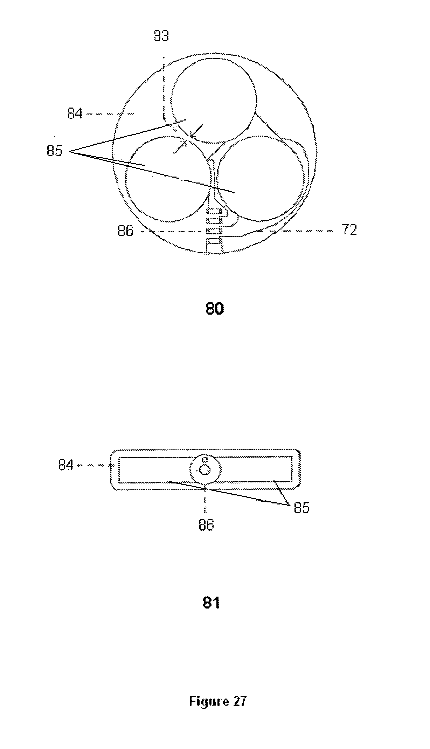

**FIG. 27.** is technical drawing of the multi-contact (four contacts/three channels) version of the new implant (top view and front view).

### BRIEF DESCRIPTION OF THE LABELS USED IN DRAWINGS

(1) Transmitter.
(2) Active coil.
(3) Passive coil.
(4) Ferrite pot core.
(5) 42 S.W.G. (standard wire gauge) enammaled copper wire.
(6) Magnetic flux.
(7) Current flowing in the coil.
(8) Therapeutic signal supplied by the transmitter device.
(9) Therapeutic signal induced at the output of passive coil.
(10) Miniature electrode implanted in epidural space of the spinal cord.
(11) Skin between active and passive coils.
(12) CMOS 556 double timer.
(13) CMOS 555 single timer.
(14) Slow signal output of the double timer.
(15) Fast signal output of the double timer.
(16) Reset terminal of the single timer.
(17) Connections of the stimülasyon modes selector switch.
(18) R1 (30 k 0.25 W metal film resistor).
(19) R2 (30 k 0.25 W metal film resistor).
(20) R3 (30 k 0.25 W metal film resistor).
(21) R4 (43 k 0.25 W metal film resistor).
(22) R5 (1.8 k 0.25 W metal film resistor).
(23) R6 (330 Ω 0.25 W metal film resistor).
(24) R7 (10 k lineer potantiometer).
(25) R8 (150 Ω 0.25 W metal film resistor).
(26) R9 (1 k metal film resistor).
(27) C 1 (0.22 µF 35 V tantalum capacitor).
(28) C2 (10 µF 16 V tantalum capacitor).
(29) C3 (0.1 µF 35 V tantalum capacitor).
(30) C4 (0.1 µF 35 V tantalum capacitor).
(31) C5 (47 µF 16 V tantalum capacitor).
(32) C6 (1 µF 100 V minik electrolytic capacitor).
(33) D1 (1N4148 diode).
(34) D2 (1N4148 diode).
(35) D3 (1N4148 diode).
(36) TRS (ZTX605 darlington transistor).
(37) TRF (8x1 amplification output transformer).
(38) MPR (mikro power regulator).
(39) 9 V direct current (D.C.) input from PP3 model battery.
(40) Output of therapeutic signal from the transmitter device.
(41) Indicator lamp (low current LED).
(42) Optional resistor in the transmitter circuit (short-circuit for neuro-implant, 5.1 Ω for percutaneous stimulation, 1 Ω for TENS application).
(43) Graphic of the results of vertical distance tests with active and passive coils.
(44) Graphic of the results of vertical distance tests with active and passive coils.
(45) Graphic of the results of vertical distance tests with active and passive coils.
(46) Graphic of the results of vertical distance tests with active and passive coils.
(47) Graphic of the results of vertical distance tests with active and passive coils.
(48) Graphic of the results of lateral distance tests with active and passive coils.
(49) Graphic of the results of lateral distance tests with active and passive coils.
(50) Technical drawing of the active coil (top view).
(51) Technical drawing of the active coil (cross section).
(52) Ferrite pot core.
(53) Active coil.
(54) Output of the connector..
(55) Technical drawing of the coil former used for active coil.
(56) Technical drawing of the coil former used for active coil (cross section).
(57) 1x4 mm soldering terminals.
(58) Technical drawing that shows the encapsulation of active coil.
(59) Technical drawing that shows the encapsulation of active coil (cross section).
(60) Encapsulation with polyuretan.
(61) Active coil.
(62) Protective sprey on the surface.
(63) Technical drawing of the passive coil (top view).
(64) Technical drawing of the passive coil (cross section).
(65) Ferrite core.
(66) Coil.
(67) Output of the coil.
(68) Technical drawing of the coil former used for passive coil -I (top view).
(69) Technical drawing of the coil former used for passive coil -I (cross section).
(70) Technical drawing of the coil former used for passive coil - II (top view).
(71) Technical drawing of the coil former used for passive coil-II (cross section).
(72) Technical drawing of the coil former used for passive coil (top view).
(73) Coil former.
(74) Output of the coil.
(75) Technical drawing that shows the top view of the encapsulation of passive coil.
(76) Technical drawing that shows the side view (I) of the encapsulation of passive coil.
(77) Technical drawing that shows the side view (II) of the encapsulation of passive coil.
(78) 0.75 mm thick silicone sheet on the base.
(79) Encapsulation with 1 mm thick medical grade silicone.
(80) Passive coil.
(81) Coil output wires.
(82) Connectors made from stainless steel tube with a diameter of 7.5 mm.
(83) Technical drawing that shows top view of three-channel version of the new implant.
(84) Technical drawing that shows the front view of three channel version of the new implant.
(85) 42 S.W.G. "standard wire gauge" enammeled copper wire.
(86) 1 mm distance between the passive coils.
(87) Encapsulation with 1 mm thick medical grade silicone.
(88) Passive coils.
(89) Connectors made from stainless steel tube with a diameter of 7.5 mm.

## Claims

1. An externally activated neuro-implant system for the transmission of therapeutic stimulating signals to an implanted electrode which is used for epidural spinal cord stimulation to control chronic pain, to treat peripheral vascular disease in the lower extremities and angina pectoris, to manage movement disorders with partial motor problems, vagus nerve stimulation to reduce the frequency and duration of epileptic seizures, phrenic nerve stimulation to do diaphramme pacing in respiratory disorders, deep brain stimulation to manage parkinson's disease, peroneal nerve stimulation to correct the dropped foot in neurological disorders, cohlear stimulation to improve hearing losses, or other implantable medical devices for therapy or recording with respect to the brain, spinal cord, nerves, muscles, bones, or other tissue or body organs, comprising:
a passive coil (3) housed in a ferrite pot core (4), that is implanted under the skin and is connected to a monopolar, bipolar, threepolar, quadripolar or any multipolar implantable electrode via insulated thin wires;
an active coil (2) housed in a ferrite pot core, that is placed on the skin overlying the implanted passive coil, and is linked to the transmitter via a flexible cable;
a transmitter device (1) with associated electronic circuitry including power source, timer-counter or microprocessor, microcontroller, amplifier and
output transformer configured to generate any programmable mode of electrostimulation pulses in accordance with the required therapy to drive the external active coil.

2. The neuro-implant system of claim 1 wherein said transmission includes the transfer of therapeutic signals produced by the transmitter outside the body by trans-dermal inductive coupling through the active and passive coils each housed in a ferrite pot core; also the transfer of recording or feedback signals from the brain, spinal cord, nerves, muscles, bones, or other tissue or body organs.

3. The neuro-implant system of claim 1 wherein said transmission further includes the transfer of recording or feedback signals from the brain, spinal cord, nerves, muscles, bones, or other tissue or body organs by trans-dermal inductive coupling through the active and passive coils each housed in a ferrite pot core.

4. The neuro-implant system of claim 1, further comprising a fully passive implanted part including only a passive coil, housed in a ferrite pot core, that is connected to an implanted electrode, thus avoiding the risk of additional surgery which may result from electronic breakdown or expired battery.

5. The neuro-implant system of claim 1, further comprising more than one passive coil, e.g. two or three, each housed in a ferrite pot core, to construct a passive coils array combined with multi-contact electrodes for switching of electrical stimulation between a number of sites along target neurons, and thereby combat electrode placement difficulties.

## Patentansprüche

1. Ein von außen aktiviertes Neuro-Implantat-System für die Transmission der therapeutischen Stimulationssignale zu einer eingepflanzten Elektrode, die für epidurale Rückenmarkanregung benutzt wird, um die chronischen Schmerzen zu kontrollieren, periphere Gefäßkrankheit in den unteren Extremitäten und Angina pectoris zu behandeln, die partialen Bewegungseinschränkungen mit motorischen Problemen zu handhaben, vagus Nervenanregung zur Reduzierung der Frequenz und Dauer von epileptischen Beschlagnahmen, phrenic Nervenanregung bei Atmungsstörungen den Schritt des Zwerchfells zu verstellen, bei geringer Gehirnanregung um Parkinson-Krankheit zu behandeln, peronealer NervenAnregung um den gefallenen Fuß in den neurologischen Störungen zu beheben, cohlear Anregung um Verluste der Hörfähigkeit zu verbessern oder anderen verpflanzbaren medizinischen Vorrichtungen für Therapie oder Aufnahme in Bezug auf das Gehirn, Rückenmark, Nerven zu handhaben, Muskeln, Knochen oder andere Gewebe- oder Körperorgane, enthält:
eine in einem Ferrittopfkern (4) angeordnete passive Spule (3), welche durch Isolierleitungen an eine monopolare, zweipolige, dreipolige, vierpolige oder jede mehrpolige verpflanzbare Elektrode angeschlossen unter die Haut eingepflanzt wird;
eine in einem Ferrittopfkern untergebrachte aktive Spule (2), die auf die Haut gesetzt wird, welche die eingepflanzte passive Spule überlagert und mit dem Übermittler über ein flexibles Kabel verbunden wird;
eine Übermittlervorrichtung (1) mit einem elektronischen Schaltkreis einschließlich Energiequelle, Dauerzähler oder Mikroprozessor, Mikrocontroller, Verstärker und der Ausgangstransformator verbunden zusammengebaut wird, um jeden programmierbaren Modus von Elektro-Stimulation zu erzeugen, pulsiert in Übereinstimmung mit der erforderlichen Therapie, um die aktive Spule extern zu aktivieren.

2. System zur Neuro-Einpflanzung nach Anspruch 1, wobei die besagte Transmission die Übertragung der therapeutischen Signale einschließt, die durch den Übermittler außerhalb des Körpers durch die aktiven und passiven Spulen, welche in einem Ferrittopfkern untergebracht sind, produziert werden, sowie die Übertragung der Aufnahme oder Rückkopplungssignale vom Gehirn, vom Rückenmark, von den Nerven, von den Muskeln, von den Knochen oder von anderen Gewebe- oder Körperorganen.

3. System zur Neuro-Einpflanzung nach Anspruch 1, wobei die besagte Transmission weiter durch die Übermittlung hautinduktiven Koppelung über die aktiven und passiven Spulen, welche in einem Ferrittopfkern umwickelt untergebracht sind, die Übertragung der Aufnahme einschließt, sowie die der Rückkopplungssignale vom Gehirn, vom Rückenmark, von den Nerven, von den Muskeln, von den Knochen oder von anderen Gewebe- oder Körperorganen.

4. System zur Neuro-Einpflanzung nach Anspruch 1, weiter ein völlig passives eingepflanztes Teil einschließlich nur einer passiven Spule, die in einem Ferrittopfkern untergebracht ist, welche an eine eingepflanzte Elektrode angeschlossen wird, so dass die Gefahr der zusätzlichen Chirurgie vermeidet werden, die aus elektronischem Zusammenbruch oder abgelaufener Batterie resultieren kann.

5. System zur Neuro-Einpflanzung nach Anspruch 1, wobei weiter mehr als eine umwickelte passive Spule, z.B. zwei oder drei, jede in einem Ferrittopfkern untergebracht ist, vorhanden, um eine passive Reihe zu konstruieren, die mit mehrpoligen Elektroden für die Schaltung der elektrischen Anregung zwischen einer Anzahl von Aufstellungsorten entlang der Zielneuronen kombiniert werden, so dass die Elektrode Platzierungsschwierigkeiten bekämpfen.

## Revendications

1. Un implant neural activé extérieurement pour la transmission de signaux de stimulation thérapeutiques à un électrode placé dans le corps comprenant :
une bobine passive (3) placée dans un pot en ferrite (4), placée sous la peau, reliée à l'aide de fils fins isolés à un électrode monopolaire, bipolaire, tripolaire, quadripolaire ou à tout électrode multipolaire pouvant se placer à l'intérieur du corps;
une bobine active (2) placée dans un pot en ferrite, reliée à l'émetteur à l'aide d'un câble flexible et placée sur la peau de façon à se trouver sur la bobine passive placée dans le corps ;
un dispositif émetteur (1) ayant le circuit électronique correspondant, comprenant une alimentation, un temporisateur/compteur ou un microprocesseur, un microcontrôleur, un amplificateur et un transpondeur de sortie configurés pour générer une pulsation d'électrosimulation programmable quelconque adaptée à la thérapie nécessaire, et ce afin de faire fonctionner la bobine active externe,
utilisés pour contrôler la stimulation de moelle épinière péridurale afin de contrôler les douleurs chroniques ; ou bien utilisés pour contrôler la stimulation du nerf vague pour le traitement des extrémités inférieures ou de la maladie vasculaire périphérale dans le cas de l'angine de poitrine et pour réduire le désordre des mouvements dans lesquels peuvent exister des problèmes moteurs partiels ainsi que pour réduire les intervalles et les durées des crises d'épilepsie ; utilisés aussi pour contrôler la stimulation du nerf phrénique afin d'assurer le contrôle de la vitesse du diaphragme dans les irrégularités respiratoires, pour contrôler la stimulation cérébrale profonde dans la maladie du Parkinson, pour contrôler la stimulation du nerf péronier afin d'améliorer le bas niveau de la base dans les maladies neurologiques, utilisés aussi pour contrôler la stimulation cochléaire afin de réduire les pertes d'ouïe ou bien utilisables dans d'autres appareils médicaux pouvant se placer dans le corps pour des raisons thérapeutiques ou d'enregistrement concernant le cerveau, la moelle épinière, les nerfs, les muscles, les os ou d'autres tissus et organes du corps.

2. Implant neural selon la revendication 1, **caractérisé en ce que** la transmission en question implique le transfert à l'extérieur du corps de signaux thérapeutiques générés par un émetteur, grâce à un couplage inductif transdermique réalisé à l'aide de bobines actives et passives se trouvant chacune dans leur pot en ferrite, ladite transmission impliquant également le transfert des signaux d'enregistrement ou de réaction provenant du cerveau, de la moelle épinière, des nerfs, des muscles, des os ou d'autres tissus et organes du corps.

3. Implant neural selon la revendication 1, **caractérisé en ce que** la transmission implique d'autre part le transfert de signaux d'enregistrement et de réaction par couplage inductif transdermique à l'aide de bobines actives et passives dont chacune se trouve dans un pot en ferrite, lesdits signaux provenant du cerveau, de la moelle épinière, des nerfs, des muscles, des os ou d'autres tissus et organes du corps.

4. Implant neural selon la revendication 1, **caractérisé en ce qu'**il comprend d'autre part une composante totalement passive placée dans le corps comprenant seulement une bobine passive se trouvant dans un pot en ferrite et reliée à un électrode placé dans le corps et réduisant ainsi le risque d'interventions chirurgicales supplémentaires pouvant avoir comme conséquence des pannes électroniques ou la fin de vie de la pile.

5. Implant neural selon la revendication 1, **caractérisé en ce qu'**il comprend également plus d'une bobine, par exemple deux ou trois, dont chacune se trouve dans un pot en ferrite pour former les bobines passives combinées avec les électrodes de contact multiples pour déclencher et arrêter la stimulation électrique entre quelques régions se situant le long des neurones choisis comme cibles.
